# EUROPÄISCHE PATENTSCHRIFT

(11) **EP 3 893 815 B1**
(45) Veröffentlichungstag und Bekanntmachung des Hinweises auf die Patenterteilung: **02.11.2022**
(21) Anmeldenummer: 19813795.2
(22) Anmeldetag: 02.12.2019
(51) Int. Cl.: A61F 2/78, A61F 2/80

(54) **ORTHOPÄDIETECHNISCHE EINRICHTUNG**
ORTHOPAEDIC APPARATUS
DISPOSITIF TECHNIQUE ORTHOPÉDIQUE

(30) Priorität: 10.12.2018 DE 102018131550
(43) Veröffentlichungstag der Anmeldung: 20.10.2021
(73) Patentinhaber: Ottobock SE & Co. KGaA, 37115 Duderstadt (DE)
(72) Erfinder: FINKE, Lars Benjamin, 37136 Landolfshausen (DE)
(74) Vertreter: Gramm, Lins & Partner Patent- und Rechtsanwälte PartGmbB
(86) Internationale Anmeldenummer: PCT/EP2019/083246
(87) Internationale Veröffentlichungsnummer: WO 2020/120187

(56) Entgegenhaltungen:
- EP-A1- 3 100 704
- CN-B- 106 667 629
- DE-A1-102014 011 373
- DE-C- 917 687
- US-A- 5 156 629

## Beschreibung

Die Erfindung betrifft eine orthopädietechnische Einrichtung mit einem Grundkörper mit einer ersten Oberfläche, die mit zumindest einem haftenden Bereich versehen ist. Die erste Oberfläche kann geschlossen ausgebildet sein.

Orthopädietechnische Einrichtungen werden häufig an einem Nutzer angeordnet, um diesen zu unterstützen oder um ein Interface oder eine Kontaktstelle zu bilden, an dem oder an der weitere Komponenten angeordnet sein können. Beispielsweise können Manschetten um eine Gliedmaße herum angelegt werden, um die Muskulatur oder Gelenke zu unterstützen. Weiterhin sind aus dem Stand der Technik sogenannte Liner bekannt, die über einen Stumpf gezogen werden, um einen Schutz des Stumpfes vor Druckstellen zu ermöglichen. An der Außenseite des Liners wird ein formstabiler Schaft angeordnet, an dem weitere Prothesenkomponenten angeordnet werden. Weiterhin existieren sogenannte Kniekappen als Dichtelemente, die auf der Außenseite eines Prothesenschaftes aufgebracht werden, den proximalen Rand überdecken und eine Abdichtung zur Erzeugung eines Unterdruckes bereitstellen.

Vielfach ist die Innenseite einer solchen orthopädietechnischen Einrichtung mit einer haftenden Oberfläche versehen oder zumindest bereichsweise mit einer haftenden Oberfläche versehen, um die jeweilige orthopädietechnische Einrichtung möglichst ortsstabil zu halten. Die Innenseite kann entweder auf der Gliedmaße unmittelbar aufliegen und ein Verrutschen oder ein Verschieben der orthopädietechnischen Einrichtung an der Gliedmaße verhindern oder an einer weiteren orthopädietechnischen Komponente anliegen, beispielsweise auf der Außenseite oder einer Innenseite einer prothetischen oder orthetischen Einrichtung anliegen, beispielsweise wenn die orthopädietechnische Einrichtung als ein Liner oder Innenschaft zwischen einem Stumpf und einem Prothesenschaft ausgebildet ist. Haftende Oberflächen können sowohl auf der Innenseite als auch auf der Außenseite der orthopädietechnischen Einrichtung angeordnet sein, um durch entsprechende Haltekräfte eine Relativbewegung der auf der Außenseite angeordneten orthopädietechnischen Komponente, beispielsweise dem Prothesenschaft, und dem Stumpf zu verhindern oder zu verringern.

Die beschriebenen Haftkräfte werden beabsichtigt aufgebracht, um Relativbewegungen zu vermeiden. Das Anziehen einer orthopädietechnischen Komponente wird dadurch jedoch deutlich erschwert. Während des Anziehvorganges sind Relativbewegungen der orthopädietechnischen Komponente notwendig, um diese in beabsichtigter Weise auf dem Körper des Anwenders zu positionieren. Da die Ausbildung von haftenden Oberflächen wesentlich für die Erzielung der gewünschten Funktion ist, nämlich eine Ortsveränderung zu vermeiden, beispielsweise um während des Gehens einen Verlust oder ein Verrutschen einer Prothese zu vermeiden, wird das mühselige Anlegen und Ablegen eines Liners in Kauf genommen. Dazu wurde die Technik des Aufrollens und des Abrollens entwickelt. Dieses Verfahren lässt nachträgliche Korrekturen nicht oder nur in einem geringen Umfang zu, sodass bei einer Korrektur der gesamte Liner wieder abgerollt und wieder aufgerollt werden muss.

Ähnliche Probleme existieren bei anderen Anwendungsbereichen von orthopädietechnischen Einrichtungen.

Die EP 3 100 704 A1 betrifft eine orthopädietechnische Einrichtung in Gestalt eines Prothesenschaftes mit einem Grundkörper mit einer ersten Oberfläche auf der Innenseite des Prothesenschaftes, die mit zumindest einem haftenden Bereich versehen ist.

Aufgabe der vorliegenden Erfindung ist es, eine orthopädietechnische Einrichtung bereitzustellen, die leicht anzulegen ist, ohne dass die Funktionalität eingeschränkt wird.

Erfindungsgemäß wird diese Aufgabe durch eine orthopädietechnische Einrichtung mit den Merkmalen des Hauptanspruches gelöst. Vorteilhafte Ausgestaltungen und Weiterbildungen der Erfindung sind in den Unteransprüchen, der Beschreibung sowie den Figuren offenbart.

Die orthopädietechnische Einrichtung mit einem Grundkörper mit einer ersten Oberfläche, die mit zumindest einem haftenden Bereich versehen ist, sieht vor, dass zumindest eine Ausnehmung in dem Grundkörper vorhanden ist, die zumindest teilweise hinter dem haftenden Bereich ausgebildet und mit einem Druckund/oder Sauganschluss versehen oder gekoppelt ist, wobei die erste Oberfläche dergestalt ausgebildet ist, um bei Anlegen eines Unterdruckes in der zumindest einen Ausnehmung zumindest teilweise in die Ausnehmung hineingesogen und/oder bei Anlegen eines Überdruckes herausgedrückt zu werden. Durch das Hineinsaugen von zumindest Teilen der ersten Oberfläche wird erreicht, dass die hineingesogenen Teile oder Bereiche nicht mehr in Kontakt mit beispielsweise der Hautoberfläche, einer Schaftinnenseite oder einer anderen Oberfläche stehen, sodass in diesem Bereich keine haftende Oberflächenabschnitte unmittelbar an der anderen Oberfläche anliegen. Der haftende Bereich wird somit außer Kontakt mit der anderen Oberfläche gebracht, sodass sich die Haftwirkung der orthopädietechnischen Einrichtung insgesamt verringert. Alternativ oder ergänzend ist es möglich, durch Anlegen eines Überdruckes den haftenden Bereich herauszudrücken und über die Ebene der ersten Oberfläche ohne Überdruck hinausstehen zu lassen. Dadurch ist es möglich, unterschiedliche Haftbedingungen einzustellen und schaltbar zu machen. Wird ein Vakuum angelegt, lässt sich die orthopädietechnische Einrichtung durch die verringerte Haftfläche leicht oder überhaupt auf einer anderen Oberfläche entlang ziehen oder schieben, beispielsweise um die orthopädietechnische Einrichtung über einen Stumpf, über ein Körperteil oder eine andere orthopädietechnische Komponente, beispielsweise einen Prothesenschaft anlegen zu können. Sind beispielsweise die haftenden Bereiche zurückgesetzt ausgebildet und dellenartig geformt, lässt sich die orthopädietechnische Einrichtung ohne Überdruck leicht anlegen und in ihrer Position korrigieren und liegt nach Anlegen eines Überdruckes fest an der jeweiligen Oberfläche an und haftet daran. Beide Effekte können auch kombiniert werden, ein Unterdruck zum Anlegen und ein Überdruck zum Verstärken der Haftkräfte. Dies ist insbesondere bei orthopädietechnischen Einrichtungen vorteilhaft, die vollumfänglich um eine andere orthopädietechnische Einrichtung oder um eine Gliedmaße herum angelegt werden oder in eine Aufnahme eingeschoben werden sollen, beispielsweise in einen umfänglich geschlossenen Prothesenschaft.

Eine Variante der Erfindung sieht vor, dass die erste Oberfläche separat von dem Grundkörper ausgebildet und daran befestigt ist. Dadurch ist es möglich, die Ausnehmungen innerhalb des Grundkörpers einfach einzubringen, beispielsweise durch Ausschneiden oder beim Urformverfahren. So ist es beispielsweise möglich, dass Bohrungen, durchgehende Schlitze oder Kanäle oder auch einzelne Vertiefungen an einer Oberfläche des Grundkörpers eingebracht oder angeordnet werden, die dann durch die erste Oberfläche abgedeckt sind, nachdem die erste Oberfläche mit dem Grundkörper verbunden wurde. Dadurch kann auf eine einfache Art und Weise ein Kanalsystem oder ein System von untereinander verbundener Ausnehmungen, wie Vertiefungen, hergestellt werden, das evakuiert oder mit Überdruck versehen und damit hinsichtlich der haftenden Bereiche schaltbar ausgestaltet werden kann.

Die eingebrachten Ausnehmungen oder Durchtritte und Kanäle sind bevorzugt mit dem Druck- und/oder Sauganschluss gekoppelt. Bei durchgängigen Ausnehmungen oder Schlitzen kann die der ersten Oberfläche gegenüberliegende Seite oder Oberfläche des Grundkörpers durch eine weitere separate Oberfläche, beispielsweise eine Beschichtung oder eine aufgebrachte Abdeckung in Gestalt einer Folie oder dergleichen abgedeckt werden, um einen Unterdruck oder Überdruck zu ermöglichen. Die Abdeckung ist luftundurchlässig oder im Wesentlichen luftundurchlässig, um ein Hineinsaugen der ersten Oberfläche in die Ausnehmungen zu ermöglichen und wird auf einer der ersten Oberfläche gegenüberliegenden Seite des Grundkörpers als eine zweite separate Oberfläche an dem Grundkörper festgelegt, die die Ausnehmungen zumindest abdeckt.

Alternativ zu einer mehrteiligen Ausgestaltung der orthopädietechnischen Einrichtung besteht die Möglichkeit, den Grundkörper und die erste Oberfläche gemeinsam auszubilden, beispielsweise im Rahmen eines additiven Fertigungsverfahrens, beispielsweise 3D-Druck oder einem ähnlichen Verfahren, mit dem es möglich ist, sowohl eine geschlossene Oberfläche als auch dahinterliegende Ausnehmungen, die über einen Sauganschluss evakuierbar oder einen Druckanschluss aufblasbar sind, auszubilden.

Eine Weiterbildung der Erfindung sieht vor, dass die gesamte erste Oberfläche haftend ausgebildet ist, sodass durch alleiniges Zurückziehen und Außerkontaktbringen nach dem Evakuieren der Ausnehmungen eine ausreichend Haftverminderung stattfindet, um eine Relativverlagerung der orthopädietechnischen Einrichtung zu einer Gliedmaße oder einer anderen Komponente zu ermöglichen. Alternativ zu einer vollflächigen, haftenden Ausgestaltung, beispielsweise durch einen entsprechenden Werkstoff der ersten Oberfläche, die als Folie oder Matte beispielsweise aus Silikon ausgebildet sein kann, besteht die Möglichkeit, dass bereichsweise eine die grundsätzliche vorhandene Haftung vermindernde Beschichtung auf der ersten Oberfläche aufgebracht ist. So kann beispielsweise durch Aufbringen einer Parylen-Beschichtung eine haftungsverminderte oder rutschige Oberfläche beispielsweise im Rahmen eines CVD-Prozesses aufgebracht werden, sodass nach dem Zurückziehen oder Hineinsaugen der ersten Oberfläche in die Ausnehmungen keine haftende Oberfläche mehr innerhalb derjenigen Ebene liegt, die ohne Unterdruck oder Evakuierung der Ausnehmungen von der ersten Oberfläche gebildet wird. Befindet sich die Beschichtung ohne Druckveränderung in ihrem Ausgangszustand nicht in eine Kontaktebene, kann durch einen Überdruck der Kontakt hergestellt und ein Anhaften bewirkt werden.

Die Haftwirkung der haftenden Bereiche kann insbesondere durch ein haftendes Material, Beschichtungen oder eine Oberflächenstrukturierung erzielt werden. In einer Weiterbildung der Erfindung kann die Haftwirkung durch einen Formschluss erzielt oder unterstützt werden, so können die haftenden Bereiche etwa ein Klett vorsehen und durch Anlegen eines Überdruckes gegen ein korrespondierendes Gegenstück gedrückt werden, das beispielsweise auf der Innenseite eines Schaftes befestigt sein kann. Auch durch die Oberflächenstrukturierung des haftenden Bereiches und eine passende Strukturierung der Kontaktfläche, beispielsweise auf der Innenseite eines Schaftes, kann die Haftwirkung verstärkt oder abgeschwächt werden.

Alternativ zu dem Beschichten mit einem haftungsvermindernden Material besteht die Möglichkeit, dass die erste Oberfläche mit einer haftungsvergrößernden Beschichtung in denjenigen Bereichen versehen wird, die oberhalb der Ausnehmungen vorhanden sind. Beispielsweise kann oberhalb der Ausnehmungen eine haftende Beschichtung aus Silikon oder dergleichen aufgebracht werden, um eine erhöhte Ortsstabilität in diesen Bereichen zu gewährleisten und eine Relativbewegung zu weiteren Komponenten oder einer Gliedmaße zu verhindern oder zu vermindern. Die haftende Beschichtung und/oder der haftende Bereich der Oberfläche kann mit den Ausnehmungen übereinstimmen, kleiner sein oder aber auch über die Ausnehmungen hinausreichen.

Eine Variante der Erfindung sieht vor, dass mehrere napfartige oder als Kanäle ausgebildete Ausbildungen in dem Grundkörper angeordnet oder ausgebildet sind, die mit dem Druck- und/oder Sauganschluss in strömungstechnischer Verbindung stehen. Dadurch ist es möglich, beispielsweise vollumfänglich Bereiche mit einer erhöhten Haftung in oder außer Kontakt zu bringen oder große Flächenanteile der orthopädietechnischen Einrichtung mit unterschiedlichen Hafteigenschaften zu versehen.

Eine Weiterbildung sieht vor, dass zumindest einer oder mehrere der napfartigen oder als Kanäle ausgebildeten Ausnehmungen in dem Grundkörper über zumindest ein Ventil mit dem Druck- und/oder Sauganschluss in strömungstechnischer Verbindung stehen, sodass nicht permanent ein Pump- oder Saugbetrieb stattfinden muss, sondern dass ein einmal eingestelltes Vakuum oder ein einmal eingestellter Unterdruck oder Überdruck in den Ausnehmungen aufrecht erhalten bleibt. Dies ist insbesondere dann der Fall, wenn das Ventil als Rückschlagventil ausgebildet ist. Bevorzugt ist das Ventil schaltbar ausgebildet, sodass auch eine Befüllung der evakuierten Ausnehmungen mit einem Medium, insbesondere Umgebungsluft, möglich ist, um eine Haftungsvergrößerung zu erreichen oder umgekehrt den Überdruck aus den Ausnehmungen abzulassen.

Wenn mehrere napfartige oder als Kanäle ausgebildete Ausnehmungen in dem Grundkörper angeordnet sind, können die bevorzugt untereinander in strömungstechnischer Verbindung stehen, sodass nur ein Druck- und/oder Sauganschluss benötigt wird, um die Ausnehmungen und Kanäle zu evakuieren oder mit einem Unterdruck oder Überdruckdruck auszustatten. Zwischen den Ausnehmungen, die in strömungstechnischer Verbindung miteinander stehen, können Drosseln oder Ventile angeordnet sein, um das jeweilige Druckniveau innerhalb der Ausnehmungen einstellen oder steuern zu können. Die Ventile können einstellbar oder schaltbar ausgestaltet sein, um Bereiche mit unterschiedlichen Drücken in den Ausnehmungen in der orthopädietechnischen Einrichtung ausbilden zu können. Es können auch mehrere Druck- und/oder Sauganschlüsse für unterschiedliche Ausnehmungen vorgesehen sein.

Eine Weiterbildung der Erfindung sieht vor, dass die Ausnehmungen sich bis zu einem Rand der orthopädietechnischen Einrichtung erstrecken, um zu verhindern, dass bei einer angelegten orthopädietechnischen Einrichtung und einem Evakuieren ein Saugeffekt stattfindet und beispielsweise eine Hautpartie mit in die Ausnehmung hineingesogen wird.

Eine Weiterbildung der Erfindung sieht vor, dass die erste Oberfläche aus einem elastischen Material hergestellt ist, sodass nach Wegfall eines Vakuums die Möglichkeit besteht, dass die erste Oberfläche aufgrund der elastischen Rückstellkräfte des Materials in die Ausgangsstellung zurückkehrt, sodass eine im Wesentlichen ebene Oberfläche oder mit Einbuchtungen versehene Oberfläche bei einem nicht angelegten Vakuum oder Unterdruck oder Überdruck durch die erste Oberfläche ausgebildet wird.

Eine Weiterbildung der Erfindung sieht vor, dass die orthopädietechnische Einrichtung als Manschette, Prothesenliner, Prothesenschaft oder Prothesenschaftkomponente ausgebildet ist. Insbesondere bei einer Ausgestaltung als umfänglich anzulegende Manschette, Prothesenliner oder Prothesenschaft ist durch die Evakuierung und das Außereingriffbringen der haftenden Bereiche ein leichtes Anlegen möglich, ohne dass nach der Aufhebung des Vakuums eine Relativbewegung der orthopädietechnischen Einrichtung an der Gliedmaße ermöglicht wird. Alternativ ist das Einsteigen bei zurückgesetzten Haftbereichen bei Normaldruck möglich und eine Relativverlagerung wird bei in Wirkkontakt befindlichen Haftbereichen bei Überdruck verhindert. Bei einer Ausgestaltung der orthopädietechnischen Einrichtung als Prothesenschaftkomponente sind in einer Weiterbildung Befestigungseinrichtungen zum reversiblen Festlegen an einem formstabilen Außenschaft vorgesehen, sodass die Prothesenschaftkomponente als flexibler und gegebenenfalls elastischer Innenschaft ausgebildet werden kann, der innerhalb eines starren Außenschaftes reversibel befestigbar ist. Mit der schaltbaren Haftverringerung und schaltbaren Haftvergrößerung durch Evakuieren oder Befüllen der Ausnehmungen ist es möglich, ohne die Prothesenschaftkomponente zu entfernen, einen starren Außenschaft mit einer Polsterung zu versehen, in die einfach eingestiegen und ausgestiegen werden kann und die gleichzeitig während der Benutzung stabil an der Gliedmaße anliegt. Die Befestigungseinrichtungen dienen zum Wechseln und gegebenenfalls Reinigen der Prothesenschaftkomponente in Gestalt eines Innenschaftes, um diesen leicht reinigen zu können. Entsprechend kann auch ein Prothesenliner einfach mit einem formstabilen Außenschaft in Eingriff gebracht werden, um den Außenschaft an dem Patienten festzulegen.

Nachfolgend wird ein Ausführungsbeispiel der Erfindung anhand der beigefügten Figuren näher erläutert. Es zeigen:
- Figur 1 -: eine perspektivische Ansicht eines Prothesenschaftes;
- Figur 2 -: eine Explosionsdarstellung einer ersten Ausführungsform;
- Figur 3 -: eine Variante der Erfindung im Ausgangszustand;
- Figur 4 -: eine orthopädietechnische Einrichtung gemäß Figur 3 mit angelegtem Vakuum; sowie
- Figur 5 -: ein Ausschnitt aus der Figur 4 in einer vergrößerten Schnittdarstellung.

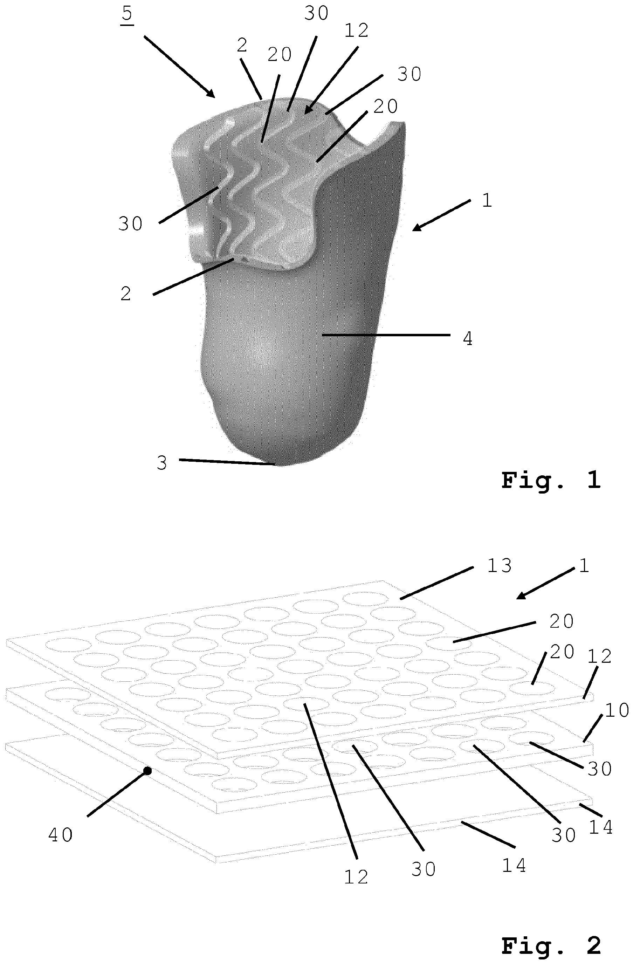

Figur 1 zeigt in einer perspektivischen Gesamtansicht einen Prothesenschaft 1 mit einer formstabilen Außenhülle 4, die eine proximale Einstiegsöffnung 5 ausbildet, die von einem proximalen Rand 2 umgeben ist. Der Prothesenschaft 1 bildet einen distalen, geschlossenen Endbereich 3 aus, an dem nicht dargestellte Befestigungseinrichtungen für eine weitere, distale Prothesenkomponente angeordnet sind. Die distale Prothesenkomponente kann eine Funktionseinheit wie ein Prothesenfuß oder eine Prothesenhand oder in einer anderen Ausführungsform ein Prothesengelenk sein. Der Prothesenschaft 1 im dargestellten Ausführungsbeispiel ist ein Unterschenkelschaft, der an einem Unterschenkelstumpf anlegbar ist.

Auf der Innenseite des Prothesenschaftes 1 ist eine orthopädietechnische Einrichtung angeordnet, entweder mit dem formstabilen Außenschaft 4 dauerhaft verbunden, beispielsweise verschweißt, verklebt oder auf andere Arten und Weisen daran befestigt oder damit ausgebildet oder über Befestigungseinrichtungen oder Befestigungselemente lösbar daran befestigt. Die orthopädietechnische Einrichtung wäre dann Teil des Prothesenschaftes und als Prothesenschaftkomponente ausgebildet, die auf der Innenseite, also auf der dem Gliedmaßenstumpf zugewandten Seite eine erste Oberfläche 12 aufweist, die mit zumindest einem haftenden Bereich 20 versehen ist. In dem dargestellten Ausführungsbeispiel sind mehrere, über den gesamten Umfang verteilt angeordnete Bereiche 20 in Zickzacklinien von dem proximalen Rand 2 bis zum distalen Endbereich angeordnet. Andere Formen sind ebenfalls möglich, insbesondere können gerade Linien oder spiralförmig angeordnete Linien ebenfalls ausgebildet und auf der ersten Oberfläche 12 angeordnet oder ausgebildet sein. Hinter den haftenden Bereichen 20 sind Kanäle 30 innerhalb eines Grundkörpers ausgearbeitet, der später erläutert werden wird. Die Kanäle 30 sind über einen nicht dargestellten Druck- und/oder Sauganschluss mit einer Pumpe oder einem Kompressor verbunden, über den Luft in die Kanäle 30 hineingepumpt oder über die Luft aus den Kanälen 30 abgepumpt werden kann. Der Anschluss für eine Pumpe oder einen Kompressor kann an dem stabilen Außenschaft 4 oder an der orthopädietechnischen Einrichtung auf der Innenseite des Außenschaftes 4 angeordnet sein.

Figur 2 zeigt eine perspektivische Explosionsdarstellung einer orthopädietechnischen Einrichtung 1 mit einem Grundkörper 10, der bevorzugt flexibel, insbesondere elastisch ausgebildet ist und einer ersten Oberfläche 12 und einer zweiten Oberfläche 14. Die ersten und zweiten Oberflächen 12, 14 sind als Folien oder Lagen aus einem entsprechenden Material ausgebildet, beispielsweise Silikon oder einem anderen Polymer. Sowohl der Grundkörper 10 als auch die beiden Oberflächen 12, 14 können eine vorgegebene Form aufweisen, die im Wesentlichen aufrecht erhalten bleibt, wenn die orthopädietechnische Einrichtung 1 genutzt wird. In dem dargestellten Ausführungsbeispiel ist die orthopädietechnische Einrichtung 1 als ein flächiges Element ausgebildet, was beispielsweise auf der Innenseite eines Prothesenschaftes angeordnet und daran dauerhaft oder reversibel befestigbar ist. Es besteht auch die Möglichkeit, dass die zweite Oberfläche 14 durch den Prothesenschaft oder eine andere Komponente gebildet wird.

An dem Grundkörper 10 ist ein Anschluss 40 für eine Saug- und/oder Druckeinrichtung, also eine Pumpe und/oder einen Kompressor angeordnet oder ausgebildet, um Luft oder ein Fluid aus Ausnehmungen 30, die in dem Grundkörper 10 ausgebildet sind, herauszupumpen oder ein Fluid in sie hineinzupumpen. Die Ausnehmungen 30 sind in dem dargestellten Ausführungsbeispiel als Durchgangslöcher ausgebildet, sie können bei dem Herstellprozess des Grundkörpers 10 ausgebildet oder nachträglich eingebracht werden, beispielsweise durch Ausstanzen, Ausschneiden oder andere Trennverfahren. Der Grundkörper 10 wird zur Fertigstellung der orthopädietechnischen Einrichtung 1 mit der zweiten Oberfläche 14 auf der Unterseite oder Außenseite des Grundkörpers 10 verbunden, beispielsweise verschweißt oder verklebt, sodass eine im Wesentlichen geschlossene Außenoberfläche entsteht. Auf der gegenüberliegenden Seite des Grundkörpers 10 wird die erste Oberfläche 12 aufgebracht, ebenfalls dergestalt, dass eine luftdichte Verbindung mit dem Grundkörper 10 vorhanden ist, beispielsweise durch an dem Umfang umlaufende oder die Ausnehmungen 30 umgebende Schweißnaht oder Klebenaht. Die einzelnen Ausnehmungen 30 können durch Verbindungskanäle in strömungstechnischer Verbindung miteinander stehen. Diese Verbindungskanäle können in dem Grundkörper 10, in der zweiten Oberfläche 14 oder in der ersten Oberfläche 12 ausgebildet sein, ebenfalls ist es möglich, mehrere strömungstechnische Verbindungen auszubilden, sodass die jeweils durch die erste Oberfläche 12, den Grundkörper 10 mit den Ausnehmungen 30 und der zweiten Oberfläche 14 gebildeten Volumina evakuiert und oder mit einem Fluid befüllt werden kann.

Auf der Oberseite der ersten Oberfläche 12, also auf derjenigen Seite, die dem Grundkörper 10 gegenüberliegt, sind haftende Bereiche 20 ausgebildet, die korrespondierend zu den Ausnehmungen 30 in dem Grundkörper angeordnet sind. In dem dargestellten Ausführungsbeispiel sind sowohl die Ausnehmungen 30 als auch die haftenden Bereiche 20 rund ausgebildet. Die Fläche und Ausdehnung der haftenden Bereiche 20 entspricht im Wesentlichen der Kontur der Ausnehmungen 30. Grundsätzlich ist es auch möglich, dass die Fläche der haftenden Bereiche 20 kleiner oder auch geringfügig größer als die Fläche der darunterliegenden Ausnehmungen 30 ist. Jenseits der haftenden Bereiche 20 ist die Oberfläche 12 auf der Innenseite glatter oder weniger haftend, insbesondere mit einer die Haftung vermindernden Beschichtung 13 versehen. Die Innenseite oder Oberseite der ersten Oberfläche 12 kann zunächst aus einem haftenden Material hergestellt werden, beispielsweise aus einem haftenden Silikon, das durch eine entsprechende Beschichtung, beispielsweise durch eine Parylenbeschichtung im Rahmen eines CVD-Verfahrens in haftende, voneinander getrennte Bereiche 20 unterteilt wird. Umgekehrt besteht die Möglichkeit, zunächst die Oberfläche 12 aus einem glatten, nicht haftenden Material herzustellen und anschließend die haftenden Bereiche 20 durch eine entsprechende Beschichtung aufzubringen.

In dem dargestellten Ausführungsbeispiel der Figur 2 sind der Grundkörper 10 und die beiden Oberflächen 12, 14 jeweils als separate Bauteile oder Komponenten ausgebildet, die aneinander festgelegt sind, um einen Hohlraum zusammen mit den Ausnehmungen 30 zu bilden. Dies hat den Vorteil eines einfachen Herstellverfahrens mit einer hochgradig variablen Fertigung.

In dem dargestellten Ausführungsbeispiel befinden sich die haftenden Bereiche 20 und die mit einer die Haftung vermindernden Beschichtung 13 versehenen Bereiche der Innenseite der ersten Oberfläche 12 in einer gemeinsamen Ebene. Wird ein Unterdruck über den Sauganschluss 40 angelegt, werden die Bereiche der ersten Oberfläche 12, die oberhalb der Ausnehmungen 30 liegen, in die Ausnehmungen 30 hineingesaugt. Ist die zweite Oberfläche 14 formstabiler als die erste Oberfläche 12, wird nur das Material der ersten Oberfläche 12 in die Ausnehmungen 30 hineingesaugt. Dadurch werden die haftenden Bereiche 20 in eine Ebene gebracht, die tiefer als die Ausgangsebene oder die Ebene der haftungsverminderten Beschichtung 30 oder unterhalb der Ebene des Grundkörpermaterials liegt, sodass die haftenden Bereiche 20 nicht mehr oder nur in einem verringerten Maße mit einer auf der dem Grundkörper abgewandten Seite der ersten Oberfläche 12 in Kontakt tretenden Körper anliegen. Dadurch ist es möglich, die Reibung zwischen der orthopädietechnischen Einrichtung 1 und dem Körper durch Anlegen eines Unterdruckes einzustellen. Umgekehrt können durch Anlegen eines Überdruckes durch Hineinpumpen von Fluid durch den Druckanschluss 40 die haftenden Bereiche 20 herausgedrückt werden, sodass diese über die Grundebene hinausstehen, um dadurch ausschließlich die haftenden Bereiche 20 in Kontakt zu bringen oder einen erhöhten Anpressdruck der haftenden Bereiche 20 an den Körper zu ermöglichen. Grundsätzlich ist es auch möglich, zunächst zurückgezogene haftende Bereiche 20, also hinter der Grundebene zurückbleibende, dellenartige oder napfartige haftende Oberflächen vorzusehen, die nur durch Anlegen eines Überdruckes in den Ausnehmungen in Kontakt mit einem aufliegenden oder anliegenden Körper treten. Bevorzugt ist das Material der ersten Oberfläche 12 elastisch oder mit einem Rückstellvermögen versehen, sodass die erste Oberfläche 12 nach Wegfall eines Überdruckes oder Unterdruckes wieder in eine Ausgangsstellung zurücckehrt.

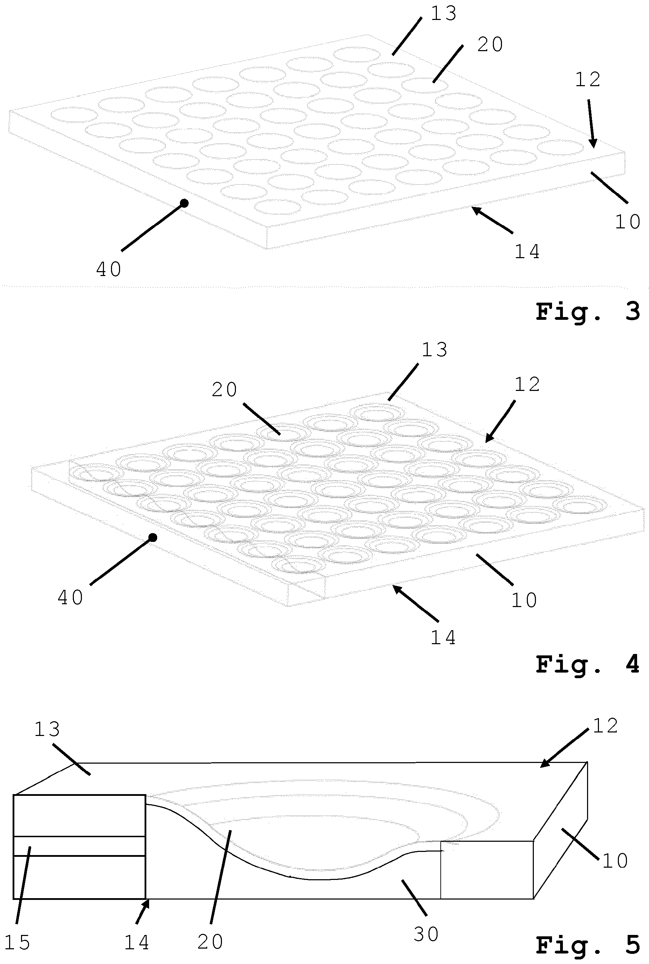

Eine Variante der Erfindung ist in der Figur 3 gezeigt, bei der statt einer mehrteiligen Ausgestaltung der orthopädietechnischen Einrichtung 1 mit einem separaten Grundkörper 10, einer separaten ersten Oberfläche 12 und einer separaten zweiten Oberfläche 14 eine einstückige Ausgestaltung gewählt ist. Das Herstellverfahren einer solchen orthopädietechnischen Einrichtung kann beispielsweise additive Komponenten aufweisen, sodass bei der Herstellung der orthopädietechnischen Einrichtung 1 sowohl die Ausnehmungen in Gestalt von Hohlräumen als auch deren strömungstechnische Verbindungen und die haftenden Bereiche 20 während des Urformverfahrens erzeugt werden. In der Figur 3 ist die orthopädietechnische Einrichtung 1 in einem Ausgangszustand gezeigt, bei der die Oberseite der ersten Oberfläche 12 im Wesentlichen eben ist.

In der Figur 4 ist die Ausführungsform gemäß Figur 3 in einem Zustand gezeigt, in dem ein Unterdruck durch den Sauganschluss 40 angelegt wurde. Die mit der haftenden Beschichtung versehenen Bereiche 20 sind in die Ausnehmungen 30 hineingezogen, sodass sich eine napfartige, eingedellte Oberflächenstruktur ergibt. Die haftenden Bereiche 20 sind jenseits der Ebene, die durch die nicht haftende oder haftungsverminderte Beschichtung 13 im Bereich des Grundkörpers 10 gebildet ist, zurückgesetzt, die zweite Oberfläche 14 ist im Wesentlichen eben geblieben.

Figur 5 zeigt eine Schnittdarstellung durch einen Teil einer orthopädietechnischen Einrichtung 1 mit dem Grundkörper 10, der daran ausgebildeten Ausnehmung 30 und den ersten und zweiten Oberflächen 12 und 14, die zusammen mit dem Grundkörper 10 ein Volumen abschließen, das durch ein Verbindungskanal 15 mit einer nicht dargestellten weiteren Ausnehmung und einem entsprechenden Volumen in strömungstechnischer Verbindung steht. In dem Zustand gemäß der Figur 5 ist die erste Oberfläche 12 mit dem haftenden Bereich 20 in die Ausnehmung 30 hineingezogen, da ein Unterdruck innerhalb der Ausnehmung 30 angelegt ist. Wird ein Überdruck angelegt oder der Unterdruck ausgeglichen, sodass innerhalb der Ausnehmung der Atmosphärendruck herrscht, kehrt die erste Oberfläche 12 in ihren Ausgangszustand zurück, sodass der haftende Bereich 20 in einer Ebene mit der übrigen ersten Oberfläche 12, insbesondere mit der Ebene, in der die nicht haftende Beschichtung 13 aufgebracht ist, liegt. In dem dargestellten Zustand ist es möglich, dass die erste Oberfläche 12 mit der Außenseite auf einem anliegenden Körper entlanggleitet, da die nicht haftende oder in ihrer Haftung verringerte Beschichtung 13 dies ermöglicht. Sobald der haftende Bereich 20 in der Ebene der nicht haftenden Beschichtung 13 oder darüber hinaus aus der Ausnehmung 30 hinausgerückt wird, tritt der haftende Bereich 20 in Kontakt mit der aufliegenden Oberfläche des Körpers und vergrößert die Haftung und verhindert eine Relativbewegung zwischen der orthopädietechnischen Einrichtung und dem weiteren Körper.

## Patentansprüche

1. Orthopädietechnische Einrichtung mit einem Grundkörper (10) mit einer ersten Oberfläche (12), die mit zumindest einem haftenden Bereich (20) versehen ist, **gekennzeichnet durch** zumindest eine Ausnehmung (30) in dem Grundkörper (10), die zumindest eine Ausnehmung (30) ist zumindest teilweise hinter dem haftenden Bereich (20) ausgebildet und mit einem Druckund/oder Sauganschluss (40) versehen, wobei die erste Oberfläche (12) ausgebildet ist, bei Anlegen eines Unterdruckes in der zumindest einen Ausnehmung (30) zumindest teilweise in die Ausnehmung (30) hineingesogen und/oder bei Anlegen eines Überdruckes herausgedrückt zu werden.

2. Orthopädietechnische Einrichtung nach Anspruch 1, **dadurch gekennzeichnet, dass** die erste Oberfläche (12) separat von dem Grundkörper (10) ausgebildet und daran befestigt ist.

3. Orthopädietechnische Einrichtung nach Anspruch 1 oder 2, **dadurch gekennzeichnet, dass** auf einer der ersten Oberfläche (12) gegenüberliegenden Seite des Grundkörpers (10) eine zweite separate Oberfläche (14) festgelegt ist, die zumindest die Ausnehmungen (30) abdeckt.

4. Orthopädietechnische Einrichtung nach einem der voranstehenden Ansprüche, **dadurch gekennzeichnet, dass** der Grundkörper (10) und die erste Oberfläche (12) einstückig ausgebildet sind.

5. Orthopädietechnische Einrichtung nach einem der voranstehenden Ansprüche, **dadurch gekennzeichnet, dass** die gesamte erste Oberfläche (12) haftend ausgebildet ist oder bereichsweise eine die Haftung vergrößernde oder vermindernde Beschichtung (13) auf der ersten Oberfläche (12) aufgebracht ist.

6. Orthopädietechnische Einrichtung nach einem der voranstehenden Ansprüche, **dadurch gekennzeichnet, dass** mehrere napfartige oder als Kanäle ausgebildete Ausnehmungen (30) in dem Grundkörper (10) angeordnet sind, die mit dem Druck- und/oder Sauganschluss (40) in strömungstechnischer Verbindung stehen.

7. Orthopädietechnische Einrichtung nach Anspruch 6, **dadurch gekennzeichnet, dass** zumindest zwei der napfartigen oder als Kanäle ausgebildeten Ausnehmungen (30) in dem Grundkörper (10) über ein Ventil mit dem Druckund/oder Sauganschluss (40) in strömungstechnischer Verbindung stehen.

8. Orthopädietechnische Einrichtung nach einem der voranstehenden Ansprüche, **dadurch gekennzeichnet, dass** mehrere napfartige oder als Kanäle ausgebildete Ausnehmungen (30) in dem Grundkörper (10) angeordnet sind, die untereinander in strömungstechnischer Verbindung stehen.

9. Orthopädietechnische Einrichtung nach Anspruch 8, **dadurch gekennzeichnet, dass** in strömungstechnischen Verbindungen zwischen Ausnehmungen (30) Drosseln oder Ventile angeordnet sind.

10. Orthopädietechnische Einrichtung nach einem der voranstehenden Ansprüche, **dadurch gekennzeichnet, dass** die Ausnehmungen (30) sich bis zu einem Rand (2) der orthopädietechnischen Einrichtung (1) erstrecken.

11. Orthopädietechnische Einrichtung nach einem der voranstehenden Ansprüche, **dadurch gekennzeichnet, dass** der haftende Bereich (20) ausschließlich oberhalb der Ausnehmungen (30) angeordnet ist.

12. Orthopädietechnische Einrichtung nach einem der voranstehenden Ansprüche, **dadurch gekennzeichnet, dass** die erste Oberfläche (12) aus einem elastischen Material hergestellt ist.

13. Orthopädietechnische Einrichtung nach einem der voranstehenden Ansprüche, **dadurch gekennzeichnet, dass** sie als Manschette, Prothesenliner, Prothesenschaft oder Prothesenschaftkomponente ausgebildet ist.

14. Orthopädietechnische Einrichtung nach Anspruch 13, **dadurch gekennzeichnet, dass** die Prothesenschaftkomponente Befestigungseinrichtungen zum reversiblen Festlegen an einem formstabilen Außenschaft aufweist.

## Claims

1. An orthopedic device comprising a main body (10) with a first surface (12) which is provided with at least one adhesive region (20), **characterized by** at least one recess (30) provided in the main body (10), said at least one recess (30) being at least partially formed behind the adhesive region (20) and being provided with a pressure and/or suction port (40), wherein the first surface (12) is designed to be at least partially sucked into the recess (30) when a negative pressure is applied in the at least one recess (30) and/or to be pushed out when a positive pressure is applied.

2. The orthopedic device as claimed in claim 1, **characterized in that** the first surface (12) is designed separately from the main body (10) and is secured thereon.

3. The orthopedic device as claimed in claim 1 or 2, **characterized in that**, on a side of the main body (10) lying opposite the first surface (12), a second, separate surface (14) is secured which at least covers the recesses (30).

4. The orthopedic device as claimed in one of the preceding claims, **characterized in that** the main body (10) and the first surface (12) are designed in one piece.

5. The orthopedic device as claimed in one of the preceding claims, **characterized in that** the entire first surface (12) is designed to be adhesive, or a coating (13) increasing or reducing the adhesion is applied to some regions of the first surface (12).

6. The orthopedic device as claimed in one of the preceding claims, **characterized in that** a plurality of cup-like or channel-shaped recesses (30) are arranged in the main body (10) and are fluidically connected to the pressure and/or suction port (40).

7. The orthopedic device as claimed in claim 6, **characterized in that** at least two of the cup-like or channel-shaped recesses (30) in the main body (10) are fluidically connected to the pressure and/or suction port (40) via a valve.

8. The orthopedic device as claimed in one of the preceding claims, **characterized in that** a plurality of cup-like or channel-shaped recesses (30) are arranged in the main body (10) and are fluidically connected to one another.

9. The orthopedic device as claimed in claim 8, **characterized in that** throttles or valves are arranged in fluidic connections between recesses (30) .

10. The orthopedic device as claimed in one of the preceding claims, **characterized in that** the recesses (30) extend as far as an edge (2) of the orthopedic device (1).

11. The orthopedic device as claimed in one of the preceding claims, **characterized in that** the adhesive region (20) is arranged exclusively above the recesses (30).

12. The orthopedic device as claimed in one of the preceding claims, **characterized in that** the first surface (12) is produced from an elastic material.

13. The orthopedic device as claimed in one of the preceding claims, **characterized in that** it is designed as a cuff, prosthesis liner, prosthesis socket or prosthesis socket component.

14. The orthopedic device as claimed in claim 13, **characterized in that** the prosthesis socket component has fastening devices for reversible fastening to a dimensionally stable outer socket.

## Revendications

1. Dispositif orthopédique comportant un corps de base (10) présentant une première surface (12) qui est pourvue d'au moins une zone adhérente (20),
**caractérisé par** au moins un évidement (30) dans le corps de base (10), ledit au moins un évidement (30) étant formé au moins partiellement derrière la zone adhérente (20) et étant pourvu d'un raccord de pression et/ou d'aspiration (40), la première surface (12) étant réalisée pour être aspirée au moins partiellement jusque dans l'évidement (30) lorsqu'une dépression est appliquée dans ledit au moins un évidement (30), et/ou pour être expulsée lorsqu'une surpression est appliquée.

2. Dispositif orthopédique selon la revendication 1,
**caractérisé en ce que** la première surface (12) est réalisée séparément du corps de base (10) et y est fixée.

3. Dispositif orthopédique selon la revendication 1 ou 2,
**caractérisé en ce qu'**une deuxième surface séparée (14) est immobilisée sur une face du corps de base (10) opposée à la première surface (12), qui recouvre au moins les évidements (30).

4. Dispositif orthopédique selon l'une des revendications précédentes, **caractérisé en ce que** le corps de base (10) et la première surface (12) sont réalisés d'une seule pièce.

5. Dispositif orthopédique selon l'une des revendications précédentes, **caractérisé en ce que** la totalité de la première surface (12) est adhérente ou **en ce qu'**un revêtement (13) augmentant ou diminuant l'adhérence est appliqué localement sur la première surface (12).

6. Dispositif orthopédique selon l'une des revendications précédentes, **caractérisé en ce que** plusieurs évidements (30) en forme de godets ou de canaux sont disposés dans le corps de base (10), lesquels sont en communication fluidique avec le raccord de pression et/ou d'aspiration (40).

7. Dispositif orthopédique selon la revendication 6,
**caractérisé en ce qu'**au moins deux des évidements (30) en forme de godets ou de canaux dans le corps de base (10) sont en communication fluidique avec le raccord de pression et/ou d'aspiration (40) par l'intermédiaire d'une vanne.

8. Dispositif orthopédique selon l'une des revendications précédentes, **caractérisé en ce que** plusieurs évidements (30) en forme de godets ou de canaux sont disposés dans le corps de base (10), qui sont en communication fluidique entre eux.

9. Dispositif orthopédique selon la revendication 8,
**caractérisé en ce que** des étranglements ou des vannes sont disposés dans des communications fluidiques entre des évidements (30).

10. Dispositif orthopédique selon l'une des revendications précédentes, **caractérisé en ce que** les évidements (30) s'étendent jusqu'à un bord (2) du dispositif orthopédique (1).

11. Dispositif orthopédique selon l'une des revendications précédentes, **caractérisé en ce que** la zone adhérente (20) est disposée exclusivement au-dessus des évidements (30).

12. Dispositif orthopédique selon l'une des revendications précédentes, **caractérisé en ce que** la première surface (12) est fabriquée en un matériau élastique.

13. Dispositif orthopédique selon l'une des revendications précédentes, **caractérisé en ce qu'**il est réalisé sous la forme d'un manchon, d'une doublure de prothèse, d'une emboîture de prothèse ou d'un composant d'emboîture de prothèse.

14. Dispositif orthopédique selon la revendication 13,
**caractérisé en ce que** le composant d'emboîture de prothèse présente des dispositifs de fixation pour l'immobilisation réversible à une emboîture extérieure solide en forme.
